# DEMANDE DE BREVET EUROPEEN

(11) **EP 3 326 601 A1**
(43) Date de publication de la demande: **30.05.2018**
(21) Numéro de dépôt: 17201807.9
(22) Date de dépôt: 15.11.2017
(51) Int. Cl.: A61G 5/14, A61G 5/10, A61G 5/12, A61B 5/02

(54) **APPAREIL DE LEVAGE CONNECTÉ**

(30) Priorité: 15.11.2016 FR 1661065
(71) Demandeur: Recfrance, 34600 Bedarieux (FR)
(72) Inventeur: JUSZKIEWICZ, Richard, 34600 BEDARIEUX (FR); JUSZKIEWICZ, Edouard, 34600 VILLEMAGNE L'ARGENTIERE (FR); MARKIDES, Emilie, 34300 AGDE (FR); MASSOL, Johan, 34600 BEDARIEUX (FR)
(74) Mandataire: Cabinet Plasseraud

(57) **Abrégé**

Appareil de levage connecté pour personne handicapée dans le but de contrôler sur place et à distance l'opération de levage de la personne. Il est constitué d'un module électronique principal (1), de deux accéléromètres (2 et 3), d'un bracelet pulsomètre (4), d'un appui tête (5), d'un guidon (6), d'un ensemble dossier (7), d'un ensemble assise (8), d'un ensemble basculant (9), d'un vérin électrique de verticalisation (10), d'un châssis (11), d'un vérin à gaz de bascule (12), d'un ensemble repose pieds (13), de deux appuis sous-rotuliens (14), de deux accoudoirs (15).

## Description

L'invention a pour objet un appareil de levage pour personne handicapée.

L'appareil suivant la présente invention trouvera plus particulièrement une application dans le but de mesurer des paramètres biologiques et/ou mécanique liés à la pathologie du patient ou à la maintenance de son équipement et d'échanger ces informations avec le praticien.

Le secteur technique de l'invention est celui de la construction des appareils automoteurs destinés à permettre le levage de personnes handicapées tout en contrôlant des paramètres biologiques et/mécaniques.

Des appareils de levage, ou verticalisateurs, sont connus dans la technique. On connait par exemple des appareils de levage pour piscine pour permettre à un patient d'aller dans l'eau d'une piscine sans prise de risque. La personne est maintenue par l'appareil et pénètre dans l'eau par un bras de levage.

Le problème rencontré par de tels appareils est la nécessité d'avoir une personne qui contrôle l'appareil pendant la descente dans l'eau de la piscine et la difficulté d'avoir des informations mécaniques et/ou biologiques pendant cette opération. La personne handicapée peut en effet avoir des paramètres biologiques et/ou mécaniques liés à sa pathologie qui changent pendant cette opération.

On connait des équipements qui permettent de maintenir des patients pendant l'opération de levage, comme des harnais de levage, des sièges de maintien, des sangles à demeure, des sangles amputation pour les patients auxquels il manque un ou plusieurs membres ou parties de membres, des sangles de bain pour aller dans l'eau.

Le problème rencontré par ces équipements est l'absence d'informations mécaniques et/ou biologiques pendant le maintien et l'absence d'informations communiquées au praticien pendant cette opération.

On connait des barres de relèvement pour personnes handicapées.

Le problème rencontré par ces barres est l'absence d'informations mécaniques et/ou biologiques pendant le relevage de la personne handicapée et l'absence d'informations communiquées au praticien pendant cette opération.

On connait des coussins releveurs d'urgence qui permet à une personne seule de relever en position assise ou debout une personne handicapée tombée à terre.

Le problème rencontré par ces coussins est l'absence d'informations mécaniques et/ou biologiques pendant le relevage de la personne handicapée et l'absence d'informations communiquées au praticien pendant cette opération.

On connait des verticalisateurs électriques qui permettent de faciliter l'opération de levage.

Le problème rencontré par ces coussins est l'absence d'informations mécaniques et/ou biologiques pendant le relevage de la personne handicapée et l'absence d'informations communiquées au praticien pendant cette opération. De plus, si le praticien n'est pas présent pendant le levage, il ne peut pas contrôler les informations mécaniques et/ou biologiques pendant le relevage de la personne handicapée.

On connait des fauteuils roulants qui peuvent permettre d'effectuer le levage de la personne handicapée tel que décrit dans FR3034985 et WO 2015071616.

Le problème rencontré par ces fauteuils roulants est l'absence d'informations mécaniques et/ou biologiques pendant le relevage de la personne handicapée et l'absence d'informations communiquées au praticien pendant cette opération. De plus, si le praticien n'est pas présent pendant le levage, il ne peut pas contrôler les informations mécaniques et/ou biologiques pendant le relevage de la personne handicapée.

On connait des logements médicalisés mobiles qui contiennent toutes les connections nécessaires permettant un raccordement aux réseaux et contenant des équipements comme des caméras de surveillance, un lit médicalisé, un capteur de température et de rythme cardiaque, un verticalisateur, un WC et douche aux normes handicapées, une climatisation réversible, un détecteur de chute, une téléassistance comme décrit dans FR3031354.

Le problème rencontré par ces logements médicalisés mobiles est l'absence d'autonomie et la difficulté de se déplacer.

Ce sont à ses problèmes que la présente invention entend remédier.

L'appareil de levage suivant l'invention a pour objectif de permettre un levage sécurisé d'une personne handicapée, tout en contrôlant les informations mécaniques et/ou biologiques pendant le relevage de la personne handicapée par un praticien présent lors de l'opération de levage ou à distance via la transmission des données vers le logiciel accessible à ce praticien.

L'appareil de levage suivant l'invention comprend un appareil de levage, une partie logicielle sous forme d'une interface de consultation web pour le praticien et un module électronique permettant de rendre un châssis thérapeutique communicant, donc apte à effectuer la mesure de paramètres biologiques et/ou mécaniques liés à la pathologie du patient ou à la maintenance de son équipement.

Les paramètres mesurés du patient peuvent être : angle d'inclinaison assise du patient, angle d'inclinaison dossier du patient, roulis du patient, tangage et lacet du châssis pour éviter le risque de basculement du patient, pulsation cardiaque du patient, fermeture de la ceinture du patient, niveau et utilisation de la batterie du fauteuil, mesure des déplacements, géolocalisation, ou tous autres paramètres techniques de l'appareil de levage et tous autres paramètres de santé du patient.

Le module électronique permet également de contrôler l'actionnement mécanique du fauteuil (électrique, pneumatique ou hydraulique) et interrompre celui-ci lorsque les mesures effectuées sortent des valeurs acceptables (par exemple, en contrôlant une opération de relèvement du patient lorsque le rythme cardiaque dépasse un seuil prédéfini).

Le module comprend une interface de communication (accès internet via wifi) permettant le reversement des informations vers le logiciel accessible au praticien.

Le module électronique est composé d'un microcontrôleur disposant d'entrées sorties binaires (GPIO, *General Purpose Input*/*Output*, littéralement Entrée/Sortie pour un Usage Général), de connectivité I2C (Inter-Integrated Circuit) pour les capteurs, ainsi que de fonctionnalités Bluetooth et Wifi ou 3G. La transmission des données depuis le module électronique au serveur se fait par le protocole MQTT (Protocole de messagerie MQ Telemetry Transport).

La consultation des informations recueillis sur l'appareil de levage se fait au travers de tableaux de bords accessibles par le web.

L'appareil de levage suivant l'invention se caractérise en ce que les paramètres de pathologie du patient présent dans l'appareil de levage et les paramètres techniques de l'appareil de levage sont mesurés par le module électronique grâce à des capteurs et transmis via une carte mère à un module logiciel connecté au praticien du patient permettant ainsi au praticien de contrôler l'opération de levage, sur place ou à distance.

La présente invention propose ainsi un appareil de levage comportant un appui tête (5), un guidon (6), un ensemble dossier (7), un ensemble assise (8), un ensemble basculant (9), un vérin électrique de verticalisation (10), un châssis (11), un vérin à gaz de bascule (12), un ensemble repose pieds (13), deux appuis sous-rotuliens (14), deux accoudoirs (15).

Dans cet appareil de levage :
Le module électronique principal (1), un accéléromètre (2), un accéléromètre (3), un bracelet pulsomètre (4) positionné sur l'avant-bras du patient permettent de mesurer les paramètres de pathologie du patient et les paramètres techniques de l'appareil de levage.

Les paramètres de pathologie du patient présent dans l'appareil de levage et les paramètres techniques de l'appareil de levage sont transmis à un module logiciel (24) par le Wifi et la 3G.

Les paramètres de pathologie du patient et les paramètres techniques de l'appareil de levage sont transmis à son praticien (27) par internet (28).

Le levage du patient est contrôlé par la praticien (27) à distance.

Les paramètres mesurés sont l'angle d'inclinaison assise du patient, l'angle d'inclinaison dossier du patient, le roulis du patient, le tangage et le lacet du châssis pour éviter le risque de basculement du patient, la pulsation cardiaque du patient, la fermeture de la ceinture du patient, le niveau et l'utilisation de la batterie du fauteuil, la mesure des déplacements, la géolocalisation.

Les paramètres mesurés sont toutes les types de paramètres mesurables concernant le patient et l'appareil de levage.

La description qui va suivre, par des figures, permettra de mieux comprendre l'invention, ses caractéristiques et ses avantages au regard de l'art antérieur.
Figure 1 : l'appareil en entier comprenant un appareil de levage, une partie logicielle et une partie électronique.
Figure 2 : Schéma du module électronique et de ses interactions.

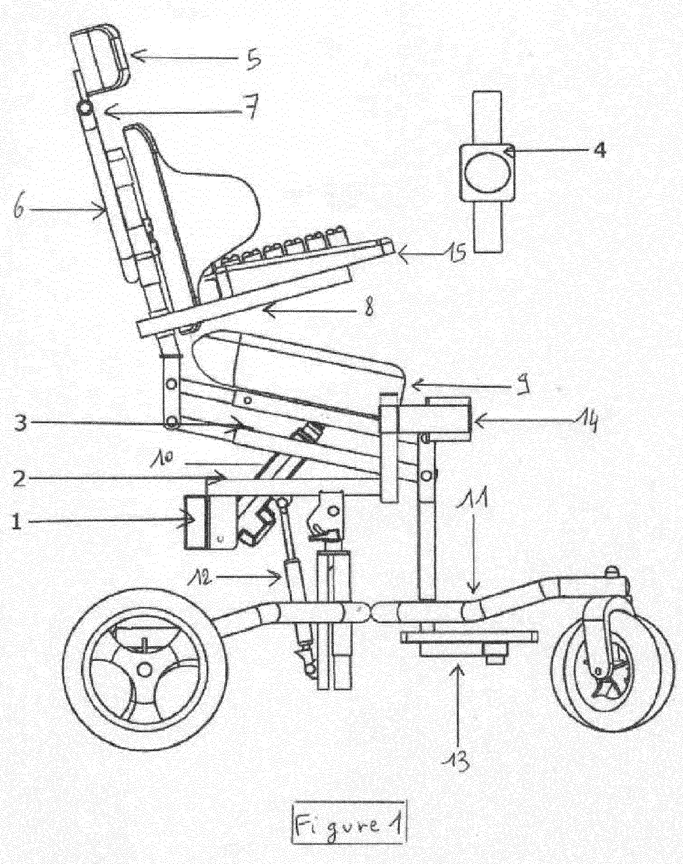

Dans la figure 1, l'appareil en entier comprend un module électronique principal (1), un accéléromètre (2), un accéléromètre (3), un bracelet pulsomètre (4), un appui tête (5), un guidon (6), un ensemble dossier (7), un ensemble assise (8), un ensemble basculant (9), un vérin électrique de verticalisation (10), un châssis (11), un vérin à gaz de bascule (12), un ensemble repose pieds (13), deux appuis sous-rotuliens (14), deux accoudoirs (15).

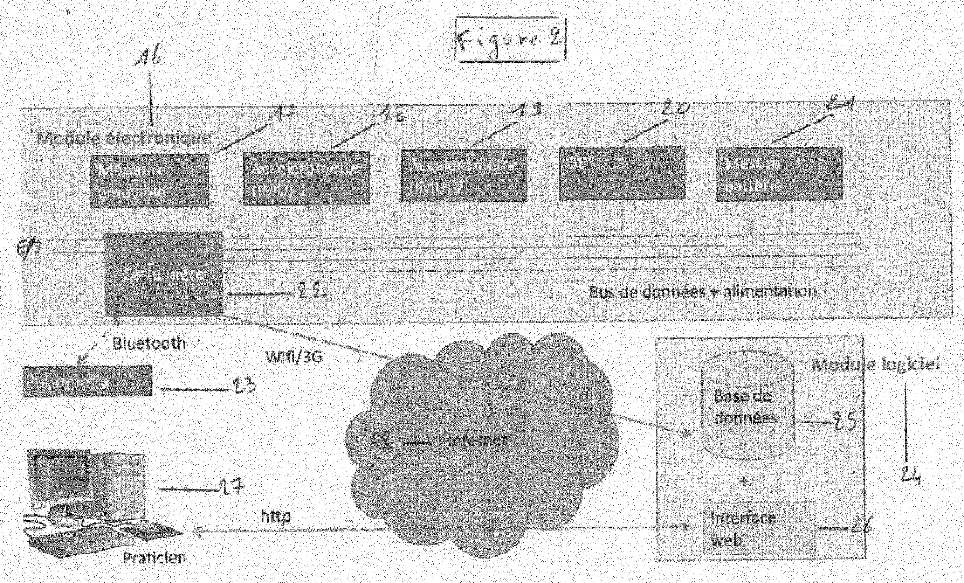

Dans la figure 2, le schéma du module électronique et de ses interactions comprend le module électronique (16) avec une mémoire amovible (17), un accéléromètre (18), un accéléromètre (19), une connexion GPS (20), une mesure de batterie (21) et une carte mère (22) ; la carte mère (22) est connectée à un pulsomètre (23) via le Bluetooth ; la carte mère (22) est connectée à un module logiciel (24) qui comprend une base de données (25) et une interface web (26) ; le module logiciel (24) est connecté au praticien (27) sur son système informatique comme un ordinateur, une tablette ou un téléphone mobile. La connexion entre la carte mère (22) et le module logiciel (24) et entre le module logiciel (24) et le praticien (27) se fait grâce à Internet (28).

## Revendications

1. Appareil de levage comportant un appui tête (5), un guidon (6), un ensemble dossier (7), un ensemble assise (8), un ensemble basculant (9), un vérin électrique de verticalisation (10), un châssis (11), un vérin à gaz de bascule (12), un ensemble repose pieds (13), deux appuis sous-rotuliens (14), deux accoudoirs (15), **caractérisé en ce que** l'appareil de levage comporte un module électronique principal (1), un accéléromètre (2), un accéléromètre (3), un bracelet pulsomètre (4) positionné sur l'avant-bras du patient qui permettent de mesurer les paramètres de pathologie du patient et les paramètres techniques de l'appareil de levage.

2. Appareil de levage selon la revendication 1, **caractérisé en ce que** les paramètres de pathologie du patient et les paramètres techniques de l'appareil de levage sont transmis à un module logiciel (24) par le Wifi et la 3G.

3. Appareil de levage selon la revendication 1 ou 2, **caractérisé en ce que** les paramètres de pathologie du patient et les paramètres techniques de l'appareil de levage sont transmis à son praticien (27) par internet (28).

4. Appareil de levage selon l'une des revendications 1 à 3 **caractérisé en ce que** le levage du patient est contrôlé par le praticien (27) à distance.

5. Appareil de levage selon l'une des revendications 1 à 4, **caractérisé en ce que** les paramètres mesurés sont l'angle d'inclinaison assise du patient, l'angle d'inclinaison dossier du patient, le roulis du patient, le tangage et le lacet du châssis pour éviter le risque de basculement du patient, la pulsation cardiaque du patient, la fermeture de la ceinture du patient, le niveau et l'utilisation de la batterie du fauteuil, la mesure des déplacements, la géolocalisation.

6. Appareil de levage selon l'une des revendications 1 à 5, caractérisé en ce les paramètres mesurés sont tous les types de paramètres mesurables concernant le patient et l'appareil de levage.
